# EUROPEAN PATENT APPLICATION

(11) **EP 4 420 878 A1**
(43) Date of publication of application: **28.08.2024**
(21) Application number: 23158337.8
(22) Date of filing: 23.02.2023
(51) Int. Cl.: B33Y 10/00, B29C 64/165, C12M 1/26

(54) **DEVICE AND METHOD FOR ACOUSTIC MANIPULATING BIOMATERIAL PARTICLES IN A FLOW OF A WORKING MEDIUM**

(71) Applicant: Max-Planck-Gesellschaft zur Förderung der Wissenschaften e.V., 80539 München (DE)
(72) Inventor: MELDE, Kai, 69120 Heidelberg (DE); FISCHER, Peer, 69120 Heidelberg (DE); SHI, Minghui, 69120 Heidelberg (DE)
(74) Representative: v. Bezold & Partner Patentanwälte - PartG mbB

(57) **Abstract**

The invention relates to a device (10) for manipulating particles (1) in a flow of a working medium (2). The device (10) comprises a mounting portion (12) for mounting the device (10) to a fluid conduit (22) for guiding the flow of the working medium (2). The device (10) further comprises an acoustic source component (14) connected to the mounting portion (12) and configured for emitting an acoustic holographic interference field (3) manipulating the particles (1) in the flow of the working medium (2). Furthermore, the invention relates to a dispenser (20) for dispensing a working medium (2) with particles (1) comprising the said device (10), and an apparatus for fabricating structures of a working medium (2) with particles (1) comprising said dispenser (20). Lastly, the invention relates to a method for manipulating particles (1) in a flow of a working medium (2).

## Description

The invention relates to a device for manipulating particles (e.g. biological cells or hydrogel beads) in a flow of a working medium (e.g. water or hydrogel). The manipulation is realized via acoustic forces of an acoustic holographic interference field. For this, the device comprises an acoustic source component (e.g. an ultrasound transducer coupled to an acoustic hologram) configured for emitting the respective acoustic holographic interference field. Furthermore, the invention relates to a dispenser for dispensing a working medium with particles comprising the said device, and an apparatus for fabricating structures of a working medium with particles comprising said dispenser. Lastly, the invention relates to a method for manipulating particles in a flow of a working medium. The method is contactless and may be, for example, performed with the device, the dispenser, and/or the apparatus mentioned before. Applications of the invention are available in the fields of fabricating materials with arbitrary shapes, additive manufacturing, rapid prototyping, and bio-fabrication. The invention can, for example, be used for assembling cell cultures to construct spheroids, organoids and/or tissues of defined shapes, composition and size. In particular, the invention may be used to form (continuous) tubular cell assemblies and/or tissue strands. The assembled particles may be organic, inorganic, or any combination thereof.

The goal of conventional bioprinting methods is to arrange biological cells on substrates. To achieve this the cells are homogeneously dispersed in a printing medium (e.g. a liquid precursor of hydrogel) to make so-called bioinks, which come in many different formulations and recipes. The most common printing technique is based on extrusion of bioink filaments from a printing nozzle, which is moved relative to a substrate during deposition. Alternative printing methods exist where a less viscous bioink is ejected in the form of droplets from an orifice through pneumatic or acoustic means (e.g. inkjet deposition). The spatial resolution of the features that can be printed with conventional bioprinters largely depends on the opening size of the printhead nozzle or orifice and there is no further control over spatial arrangement of the cells within a filament or droplet.

To overcome some of the aforementioned deficiencies, ultrasound directed self-assembly approaches are known in the prior art, see e.g. D.V. Deshmusk et al. "Continuous Production of Acoustically Patterned Cells Within Hydrogel Fibers for Musculoskeletal Tissue Engineering", Adv. Funct. Mater. 2022, 32, 2113038), to acoustically pattern biological cells in a liquid hydrogel precursor flowing through a microchannel. However, this method uses standing waves, which only allows focusing of cells into simple patterns of lines or sheets. The patterns' shape and periodicity depend on the channel geometry and the ultrasound frequency, with very limited variability or control. For example, in a cylindrical channel (circular crosssection), particles of positive acoustic contrast (basically all solid material) against water, will arrange in concentric circles. For an acoustic wavelength λ, the radii of these circles are fixed to approximately r = 0.38X + n*X/2, where r = 0 is the center of the channel and n refers to the nth channel. These correspond to zeros of the Bessel function of the first kind and zeroth order. To establish a standing wave field, the circular channel diameter is further fixed to coincide with the pressure antinodes, which correspond to extrema of the Bessel function of the first kind and zeroth order. Those are located approximately at R = 0.6λ + M*X/2, with M being an integer. It is apparent that the size of a single circle is bound to the overall diameter of the channel.

Accordingly, it is an object of the invention is to provide an improved solution for manipulating particles dispersed in a printing/working medium, wherein disadvantages and limitations of conventional techniques are avoided. In particular, the solution should allow for contactless assembling particles in a printing/working medium in arbitrary shapes with high precision.

These objectives are solved by a device, a dispenser, an apparatus, and a method, comprising the features of the independent claims, respectively. Preferred embodiments and applications of the invention result from the dependent claims.

According to a first general aspect of the disclosure, a device is provided for manipulating particles, preferably microparticles, in a (e.g. continuous) flow of a working medium (e.g. water and/or hydrogel). The particles may be organic (e.g. comprising biological cells, bacteria, organoids, tissue samples, hydrogels, and/or natural or synthetic polymers including, but not limited, to collagen, gelatin, silk, agarose, chitin, hyaluronan, alginate, dextran, cellulose, poly(caprolactone), poly(lactic acid), or polystyrene), inorganic (e.g. comprising glass, ceramics and/or metal), or any combination thereof. The particles may further be composites of different materials or hydrogel beads or droplets, which further contain smaller particles inside.

The device comprises a (e.g. sleeve-like) mounting portion for mounting the device to a (e.g. circular and/or closed) fluid conduit for guiding the flow of the working medium. The fluid conduit may be, for example, a fluid pipe and/or fluid tube (e.g. such as from a syringe needle and/or a pipette tip). The mounting portion may form an annular opening for inserting the fluid conduit therein (e.g. for providing a tight fit between the device and the fluid conduit).

The device further comprises an acoustic source component connected to the mounting portion. For example, the acoustic source component and the mounting portion may be firmly attached to each other, preferably such that the respective components cannot be separated from each other in a non-destructive way. In this context, it is also possible that the mounting portion may be formed by a segment (e.g. an outer surface segment) of the acoustic source component.

The acoustic source component is configured for emitting an (e.g. stationary) acoustic holographic interference field, manipulating (e.g. assembling) the particles in the flow of the working medium. The term "acoustic holographic interference field" may, thereby, refer to an acoustic field, which is generated by interference of several sub-waves (in a holographic process). The sub-waves may be created e.g. by at least one acoustic diffraction element (e.g. a phase plate and/or hologram of the acoustic source component) and/or an acoustic array device, i.e. an integrated array of acoustic sources. Consequently, the acoustic holographic interference field (the acoustic source component is configured to emit) is preferably not a simple plane or spherical wave sound field, but rather comprises wave fronts with a patterned/structured phase and/or amplitude distribution. In other words, the acoustic holographic interference field may comprise wave fronts with a (spatially) patterned/structured phase and/or amplitude distribution. Preferably, the pattering/structuring of the phase distribution is such that the acoustic holographic interference field has a predefined structure in an assembling portion distant from the acoustic source component. Said structure may thereby depend on the way the particles should be manipulated and/or assembled. In particular, the acoustic holographic interference field is preferably independent of the channel geometry of the fluid conduit, such that advantageously arbitrary shapes of agglomerates can be assembled. In contrast to standing wave approaches, the device advantageously allows for the creation of complex particle structures within the flow of the working medium, irrespective of the symmetry and geometry of the fluid conduit the device is mounted to. A further advantage of the claimed solution is that it is suitable for retrofitting existing systems (e.g. bioprinting systems), for example, by simply placing the device onto an existing fluid conduit of the system.

The acoustic holographic interference field may have a distribution of acoustic pressure extrema (acoustic pressure maxima and/or acoustic pressure minima). In the acoustic holographic interference field, the particles may be moved by the effect of acoustic forces. Depending on the properties (e.g. compressibility, absorptivity, and/or reflectivity) of the particles and/or the working medium, different acoustic forces may act as translation forces onto the particles. As a consequence, each particle (e.g. in a mixture or suspension) may move in a direction, which is determined by a gradient of the energy, and once it has moved to an energetically more favourable location, it can be held in this location or in the neighbourhood thereof. Accordingly, the particles may be moved towards the energy extrema of the acoustic holographic interference field. Preferably, the particles are collected around energy extrema of the acoustic holographic interference field (accumulation at the energy extrema) or at a holding surface, which is crossed by the gradients of energy provided by the acoustic holographic interference field. The holding surface can be a plane or curved surface. As an example, particles with a compressibility, which is lower than the compressibility of the working medium, may be moved to the portions of the acoustic holographic interference field, which have minimum pressure variations (e.g. pressure nodes). Otherwise, particles with a compressibility, which is larger than the compressibility of the working medium, may be moved to portions of the acoustic holographic interference field, which have maximum pressure variations. By this, movement and agglomeration of the particles may take place, wherein a shape of the resulting (one-, two-, or three-dimensional) agglomerate is determined by the acoustic holographic interference field.

According to a first aspect of the disclosure, the acoustic source component may comprise at least one (e.g. frequency-tunable) ultrasonic transducer (e.g. a piezoelectric ultrasonic transducer). The acoustic source component may, however, also comprise several (e.g. two, three, or four) ultrasonic transducers. These (e.g. frequency-tunable) several ultrasonic transducers may be arranged (e.g. concentrically) around the assembling portion and/or mounting portion (e.g. on a (imaginary) circle around the assembling portion). Preferably, the assembling portion may be located in a centre of the circle.

According to another aspect, the acoustic source component may comprise an acoustic diffractive element (e.g. an acoustic hologram, in particular an acoustic transmission hologram). In this context, the term "acoustic diffractive element" may preferably refer to a material, which is capable of interacting with a wave-front of a primary acoustic wave (e.g. emitted from the at least one ultrasonic transducer) such that the phases and/or amplitudes across the wave-front are changed by diffraction in a predetermined way. In detail, upon incident of the primary acoustic wave (e.g. a plane or spherical primary acoustic wave) on the acoustic diffractive element, the acoustic diffractive element may be configured for generating several acoustic sub-waves interfering with each other. Thereby, each changed phase and/or amplitude may provide a sub-wave contribution for the subsequent interference. Consequently, the acoustic holographic interference field may be generated via self-interference of a primary acoustic wave. In this context, the at least one ultrasonic transducer may be configured for emitting such a primary acoustic wave.

The acoustic diffractive element may be, for example, a (e.g. static) acoustic hologram (e.g. an acoustic), a (e.g. dynamic) spatial acoustic modulator (e.g. a microbubble array), and/or an acoustic (e.g. labyrinthine) metamaterial. The structure of the acoustic diffractive element for generating the (desired) acoustic holographic interference field may be calculated using an algorithm for computer-based calculation of holograms, as it is known from optics, e.g. with the Gerchberg-Saxton-Algorithm. The calculation can readily be adapted to acoustics. The use of acoustic diffractive elements has particular advantages with regard to a simplified structure of the apparatus.

In this context, in particular in the case of an acoustic hologram, the acoustic diffractive element may comprise at least two diffractive layers for determining a shape of the acoustic holographic interference field. For example, the at least two diffractive layers may have different acoustic properties, e.g. a propagation of sound and/or a speed of sound may differ in the respective layers. The at least two diffractive layers may be bonded to each other, forming an interface region. For example, the at least two diffractive layers may be arranged (e.g. concentrically) adjacent to each other. Preferably, the acoustic properties of the at least two diffractive layers and the shape of the interface determine how the primary acoustic wave is shaped, and thus the shape of the acoustic holographic interference field.

According to another aspect, the acoustic source component may comprise a (e.g. dynamic) phased array transducer. For example, the phased array transducer may comprise an (e.g. integrated) array of acoustic sources (e.g. piezoelectric oscillators), which are preferably individually controllable and each of which being adapted for emitting an acoustic wave. By individually controlling the respective acoustic sources of the array sub-waves may be emitted, which are superimposed (within the phased array transducer) and interfere with each other for generating the acoustic holographic interference field. In this context, the acoustic source component may preferably further comprise a control unit configured to control the phased array transducer to emit the acoustic holographic interference field. For example, the control unit may be configured to individually control each acoustic source of the phased array transducer such that a predetermined acoustic holographic interference field is emitted. The use of phased array transducers advantageously allows for flexible creating different acoustic holographic interference fields, without the necessity of hardware changes in the device.

According to another aspect, the mounting portion may be configured for being mounted onto the fluid conduit. For example, the mounting portion may be configured for accommodating and/or fit around a fluid pipe and/or fluid tube. Preferably, the (whole) device may be configured for being mounted onto the fluid conduit. For example, the device may be configured self-supporting, e.g. such that it can be placed, clamped, and/or pressed onto a pipe and/or tube.

In addition or alternatively, the mounting portion may form an annular opening for inserting the fluid conduit therein. The opening may, for example, be circular or oval and/or have another circumferentially closed shape. Preferably, the mounting portion is configured for the fluid conduit being (e.g. tightly) inserted in the mounting portion.

In addition or alternatively, the mounting portion may be configured for (e.g. concentrically) surrounding an outer surface of the fluid conduit. For example, the mounting portion may be configured for abutting the outer surface of the fluid conduit.

In addition or alternatively, the mounting portion may form a tapered (e.g. conical) and/or narrowing recess for accommodating the fluid conduit, preferably by clamping the fluid conduit. In other words, the mounting portion may form a recess with varying cross-section. In total, all the aforementioned embodiments advantageously enable a safe and reliable fixation of the device and/or the mounting portion to the fluid conduit.

According to another aspect, the device may comprise a (e.g. flexible) sealing element arranged at the mounting portion. For example, the sealing element may comprise a deformable pad or film (e.g. a rubber film). Preferably, the sealing element is for sealing a joint between the fluid conduit and the mounting portion. Advantageously, this enables a secure and/or tight fixation of the fluid conduit in the mounting portion.

In addition or alternatively, the device may comprise a (e.g. movable) locking element (e.g. a bracket, hook, clamp, and/or strap) for securing the device to the fluid conduit. The locking element may be configured to form a positive and/or non-positive connection with the fluid conduit. Preferably, the locking element is for preventing an unintentional removal of the device from the fluid conduit. Advantageously, this provides for a secure connection between the device and the fluid conduit.

According to another aspect, the acoustic source component may comprise a cylindrical and/or tubular ultrasonic transducer (e.g. a piezo tube actuator). Preferably, the ultrasonic transducer may thus comprise an active element for generating mechanical vibrations, which has a tubular, hollow-cylindrical and/or any other hollow-like shape, preferably comprising a constant cross-section along its longitudinal axis.

In addition or alternatively, the acoustic source component may comprise a (e.g. central) through hole. Thereby, the through hole may be formed by the at least one ultrasonic transducer and/or the acoustic diffractive element. In a preferred embodiment, the acoustic diffractive element (e.g. an acoustic hologram) - which preferably is concentrically surrounded by the at least one ultrasonic transducer - may have a tubular, hollow-cylindrical and/or any other hollow-like shape, whose inner surface defines and/or forms the respective through hole. The through hole may, for example, have a constant cross-section. Alternatively, the through hole may also have a varying (e.g. tapered) cross-section. Preferably, the through hole is for passing the flow of the working medium through the acoustic source component. Thereby, the flow of the working medium may be guided directly by the through hole itself or the fluid conduit may be accommodated within the through hole.

In addition or alternatively, the acoustic source component may comprise a concave, cylindrical, and/or (e.g. circumferentially) closed emitter surface. Generally, the term "emitter surface" may preferably refer to a surface of the acoustic source component from which the acoustic holographic interference field is emitted and/or starts to propagate. For example, in the case that the acoustic source component comprises an acoustic diffractive element (e.g. an acoustic hologram) for generating the acoustic holographic interference field, the emitter surface may be regarded as the surface of the acoustic diffractive element orientated to the assembling portion.

In addition or alternatively, the acoustic source component may be arranged for emitting the acoustic holographic interference field in a direction perpendicular to a flow direction of the flow of the working medium. For example, the acoustic source component may be arranged for emitting the acoustic holographic interference field radially with respect to the flow direction and/or radially towards a centre of the fluid conduit, when the device is mounted to the fluid conduit. Advantageously, all the aforementioned embodiments advantageously provide an easy to manufacture and accordingly cost-effective solution for fluid tubes and/or fluid pipes with constant cross-section.

In order to advantageously provide a solution in particular suitable for tapered (e.g. conical) fluid conduits (e.g. in the case of pipette tips) and/or to advantageously generate a (retarding) force component directed against the flow of the working medium, according to another aspect, the acoustic source component may comprise a conical and/or frustum-like ultrasonic transducer (e.g. a truncated conical shell with a piezoelectric layer). Preferably, the ultrasonic transducer may thus comprise an active element for generating mechanical vibrations, which has a hollow-like shape comprising a (e.g. central) hole having, which has a varying (e.g. narrowing) cross-section along the longitudinal axis of the active element.

In addition or alternatively, the acoustic source component may comprise a conical and/or tilted emitter surface. For example, the acoustic source component may be tilted with respect to an axial axis of the acoustic source component. Furthermore, the emitter surface may, for example, be concave and/or (e.g. circumferentially) closed.

In addition or alternatively, the acoustic source component may be arranged for emitting the acoustic holographic interference field tilted with respect to a flow direction of the flow of the working medium. For example, the acoustic source component may be arranged for emitting the acoustic holographic interference field at least partially along or parallel to the flow direction and/or at least partially along or parallel to the longitudinal axis of the fluid conduit, when the device is mounted to the fluid conduit. In addition or alternatively, the acoustic source component may also be arranged such that the acoustic holographic interference field provides an (e.g. retarding) acoustic force component orientated directed against the flow direction of the flow of the working medium. Notwithstanding the foregoing, the acoustic source component may also be arranged for emitting the acoustic holographic interference field at least partially radially with respect to the flow direction and/or at least partially radially towards a centre of the fluid conduit, when the device is mounted to the fluid conduit. As mentioned before, the aforementioned embodiments advantageously provide, in particular, a proper solution for fluid tubes and/or fluid pipes with tapered cross-section.

According to another aspect, the acoustic source component and/or the at least one ultrasonic transducer may comprise at least one (e.g. non-curved) ultrasonic plate transducer. The at least one ultrasonic plate transducer may, for example, have a flat and/or planar (e.g. circular, square or rectangular) emitter surface. Preferably, the acoustic source component and/or the at least one ultrasonic transducer may comprise at least two (e.g. non-curved) ultrasonic plate transducers. These at least two ultrasonic plate transducers may, for example, be arranged facing each other, i.e. parallel to each other. Alternatively, the at least two ultrasonic plate transducers may be arranged perpendicular, i.e. with an angle of 90°, to each other. Preferably, the at least two ultrasonic plate transducers are arranged at the same distance from the (common) assembling portion. According to an embodiment, the acoustic source component may further comprise an acoustic diffractive element (e.g. an acoustic transmission hologram) coupled to the at least one ultrasonic plate transducer or the at least two ultrasonic plate transducers. For example, the acoustic diffractive element may have a tubular and/or hollow-like shape with an (e.g. radial) outer surface and an (e.g. radial) inner surface, wherein the latter delimiting a (e.g. central) through hole, preferably for passing the flow of the working medium through the acoustic diffractive element. The at least one ultrasonic plate transducer or the at least two ultrasonic plate transducers may be arranged at the (e.g. radial) outer surface, preferably equidistant from the (radial) inner surface. As ultrasonic plate transducers are easy to manufacture, this aspect advantageously provides a cost-effective solution for proving a respective acoustic source component.

According to another aspect, the device may further comprise a (e.g. straight or conical) channel portion. Preferably, the channel portion is configured for receiving and guiding the flow of the working medium. For example, the channel portion may be tubular and/or cannula-like. Preferably, the channel portion may be (e.g. coaxially) surrounded by the acoustic source component and/or the acoustic diffractive element of the acoustic source component. For example, the assembling portion may be located in the channel portion, e.g. in the (radial) centre of the channel portion. In addition or alternatively, the channel portion may be at least partially formed by a (e.g. inner) surface of the acoustic source component. For example, the acoustic source component and/or the acoustic diffractive element of the acoustic source component may comprise a (e.g. central) through hole, which at least partially delimits and/or forms the channel portion. In addition or alternatively, the channel portion may be fluidically connectable to the fluid conduit. For example, the mounting portion may be arranged at an end of the channel portion, such that when the fluid conduit is mounted to the mounting portion, the channel portion is fluidically connected to the fluid conduit and/or a fluid connection between the channel portion and the fluid conduit is formed. Advantageously, the channel portion increases the flexibility of guiding the flow of the working medium within the device.

According to another aspect, the device may comprise a further (e.g. second) channel portion. Preferably, the further channel portion is configured for receiving and guiding a further flow (e.g. a second flow) of a further working medium (e.g. a second working medium). For example, the further or second working medium may be different from that flowing in the aforementioned channel portion, which may be referred to as first channel portion in this context. The further working medium may, for example, comprise further particles (e.g. second particles). Further, the device may comprise a (common) merging portion, where the further (second) channel portion joins the (first) channel portion. In other words, the (common) merging portion may provide a fluid connection between the channel portion and the further channel portion, preferably for merging and/or mixing the flow of the working medium with the further flow of the further working medium. Advantageously, this enables the formation of complex composite media, e.g. by combining working media and/or particles with different properties.

According to another aspect, the acoustic source component may be configured to (e.g. circumferentially and/or concentrically) surround the merging portion. For example, the merging portion may be arranged within the acoustic source component, e.g. in a (radial) centre of the acoustic source component. Alternatively, the merging portion may, however, also be arranged separately from the acoustic source component, e.g. upstream of the acoustic source component with respect to the flow of the working medium.

In addition or alternatively, the acoustic source component may be configured to focus the acoustic holographic interference field into the merging portion. For example, the assembling portion may thus be located within the merging portion.

In addition or alternatively, the merging portion may have an increased cross-section compared to the channel portion and the further channel portion. For example, the merging portion may have a cross-section that corresponds to the sum of the cross-section of the channel portion and the cross-section of the further channel portion.

According to another aspect, the acoustic the device may further comprise a further (e.g. second) mounting portion for mounting the device to a further (e.g. second) fluid conduit (e.g. a fluid pipe and/or fluid tube). The further channel portion may preferably be fluidically connectable to the further fluid conduit. For example, the further mounting portion may form an annular opening for inserting the further fluid conduit therein (e.g. for providing a tight fit between the device and the further fluid conduit). In this context, the further mounting portion may generally comprise any of the features described therein in the context of the mounting portion (wherein the fluid conduit is replaced by the further fluid conduit).

In addition or alternatively, the device may further comprise a fluid control component (e.g. comprising one or more switching and/or control valves). For example, the fluid control component may comprise valves (e.g. switching and/or control valves), wherein each valve is associated with one of the channel portions and the further channel portion, respectively. Preferably, the fluid control component is configured to selectively allow or disallow a fluid flow in the channel portion and the further channel portion and/or to selectively increase or decrease a fluid flow rate in the channel portion and the further channel portion. For example, the fluid control component may be configured for allowing only fluid flow in the channel portion (i.e. the fluid flow in the further channel portion is shut off) and/or for allowing only fluid flow in the further channel portion (i.e. the fluid flow in the channel portion is shut off). In addition or alternatively, the fluid control component may be configured for increasing fluid the fluid flow rate in the channel portion and decreasing the fluid flow rate in the further channel portion (or vice versa), wherein preferably the sum of both fluid flow rates is kept constant. Advantageously, this allows for a demand-based and/or efficient management of the fluid flows within the device.

In addition or alternatively, the device may further comprise an (e.g. nozzle-like) outlet and a dispensing channel portion fluidically connecting the merging portion to the outlet. In case the merging portion is arranged separately from the acoustic source component, preferably, the acoustic source component may be configured to (e.g. circumferentially and/or concentrically) surround the dispensing channel portion and/or to focus the acoustic holographic interference field into the dispensing channel portion.

In addition or alternatively, the device may further comprise another (e.g. third) channel portion configured for receiving and guiding another (e.g. third) flow of another (e.g. third) working medium. For example, the other or third working medium may be different from those flowing in the (e.g. first) channel portion and the further (e.g. second) channel portion. Preferably, the other working medium may comprise other particles (e.g. third particles). In addition or alternatively, the other channel portion joins the merging portion. For example, the (common) merging portion may provide a fluid connection between the channel portion, the further channel portion and the other channel portion, preferably for merging and/or mixing the respective flows. Advantageously, this enables the formation of complex composite media, e.g. by combining working media and/or particles with different properties.

According to a further general aspect of the disclosure, a dispenser (e.g. a print head) is provided. Preferably, the dispenser is for dispensing a working medium (e.g. hydrogel) with particles (e.g. microparticles, like biological cells, gel beads, and/or bacteria) therein. The dispenser comprises a fluid conduit (e.g. a fluid pipe and/or fluid tube) for guiding a flow of the working medium (with the particles). Preferably, the fluid conduit is orientated along the direction of gravity, i.e. vertically.

Further, the dispenser comprises a device as described herein. Accordingly, the features described in connection with the device shall also be disclosed and claimable in connection with the dispenser. The same applies vice versa. The device is, thereby, mounted to (e.g. onto) the fluid conduit, preferably for manipulating the particles in the flow of the working medium.

According to an aspect, the mounting portion may have an inner contour, which is adapted to an outer contour of the fluid conduit. Preferably, the mounting portion and the fluid conduit may thus be configured to correspond to each other and/or may be connected in a form-fit manner. Advantageously, this enables a secure and/or tight connection between the mounting portion and the fluid conduit.

In addition or alternatively, the mounting portion is mounted (e.g. only) to an end portion of the fluid conduit. For example, the end portion may be inserted into the mounting portion, which e.g. may form part of an inlet of the device. Alternatively, the mounting portion may be mounted onto the fluid conduit, e.g. such that the fluid conduit ranges through the entire mounting portion. For example, the mounting portion may be arranged onto the fluid conduit (e.g. onto its outer surface) in a belt-like or sleeve-like manner.

In addition or alternatively, the dispenser may further comprise a (e.g. fluid) acoustic coupling layer arranged between the mounting portion and the fluid conduit. For example, the acoustic coupling layer may comprise water, glycerol, and/or ethylene glycol. Preferably, the acoustic coupling layer is for transmitting sound waves between the mounting portion and the fluid conduit and/or for avoiding (e.g. air-filled) cavities (e.g. with poor sound conduction) between the mounting portion and the fluid conduit.

In addition or alternatively, the dispenser may further comprise an extruder component for generating and/or providing the flow of the working medium (with the particles). For example, the extruder component may comprise a plunger, piston and/or a conveying screw. The extruder component may be configured for (e.g. mechanically) pushing and/or transporting the working medium (with the particles) through the fluid conduit. E.g. via the extruder component, the dispenser may be configured for (e.g. metered) discharging the working medium (with the particles). In addition or alternatively, the dispenser may further comprise a pressurizing component for generating and/or providing a (e.g. positive) pressure (e.g. a gas pressure and/or biasing fluid pressure) on the working medium (with the particles). For example, the pressurizing component may be configured for pressurizing the working medium (with the particles) with a gas pressure and/or biasing fluid pressure for generating the flow of the working medium (with the particles). For this, the pressure component may e.g. comprise a pressurized gas compartment and/or a pressure regulator. The pressure component may be configured for (e.g. pneumatically and/or hydraulically) pushing and/or transporting the working medium (with the particles) through the fluid conduit.

According to a further general aspect of the disclosure, an apparatus (e.g. a 3D printer) is provided. Preferably, the apparatus is for fabricating structures of a working medium (e.g. hydrogel) with particles (e.g. microparticles, like biological cells, gel beads, and/or bacteria) on a substrate (e.g. a sample carrier). The apparatus comprises a dispenser as described herein, preferably for dispensing the working medium with particles. Accordingly, the features described in connection with the dispenser shall also be disclosed and claimable in connection with the apparatus. The same applies vice versa. Further, the apparatus comprises a positioning device configured for changing a relative position of the dispenser to the substrate, preferably in three spatial directions. In this connection, the changing of the relative position may comprise the movement of the dispenser relative to a fixed substrate, the movement of the substrate relative to a fixed dispenser and also the movement of both components relative to each other. Preferably the positioning and/or the dispensing of the working medium, which in this connection may also be referred to as printing medium or ink, is controlled by a control device of the apparatus. Thus, the apparatus may preferably further comprise a respective control device for controlling the positioning device and/or the dispenser.

According to a further general aspect of the disclosure, a method is provided for manipulating particles (e.g. microparticles) in a (e.g. continuous) flow of a working medium (e.g. water and/or hydrogel). The particles may be organic (e.g. comprising biological cells, bacteria, organoids, tissue samples, hydrogels, gel beads, and/or polymers), inorganic (e.g. comprising glass, ceramics and/or metal), or any combination thereof.

Preferably, the method is performed by using a device, dispenser, and/or apparatus described herein. Accordingly, the features described in connection with these components shall also be disclosed and claimable in connection with the method. The same applies vice versa.

The method comprises providing (e.g. generating) the flow of the working medium (e.g. by an extruder component). Preferably, the flow of the working medium (with the particles) may be provided in a fluid conduit (e.g. a fluid pipe and/or fluid tube).

The method further comprises emitting an (e.g. stationary) acoustic holographic interference field (e.g. by an acoustic source component) to the flow of the working medium for manipulating (e.g. agglomerating) the particles in the working medium. As outlined before, the acoustic holographic interference field preferably does not refer to a simple plane or spherical wave sound field, but is rather generated by interference of several sub-waves (e.g. in a holographic process). The acoustic holographic interference field may, for example, define a shape of an agglomerate to which (parts of) the particles should be assembled. The acoustic pressure field may, therefore, have a configuration/pattern, which is (set) such (e.g. calculated based on the Gerchberg-Saxton-Algorithm) that it corresponds to that of the agglomerate to be assembled. Preferably, the acoustic holographic interference field has a frequency of at least 20 kHz, in particular at least 40 kHz, up to the GHz-range.

Preferably, the method further comprises assembling the particles to an agglomerate via the acoustic holographic interference field. This may comprise moving the particles by the effect of acoustic forces of the acoustic holographic interference field. As outlined in detail before, depending on the properties (e.g. compressibility, absorptivity, and/or reflectivity) of the particles and/or the working medium different acoustic forces may act as translation forces onto the particles. As a consequence, each particle may move in a direction, which is determined by a gradient of the energy, and once it has moved to an energetically more favourable location, it can be held in this location or in the neighbourhood thereof. Accordingly, the step of assembling may comprise moving the particles towards energy extrema of the acoustic holographic interference field or to a holding surface, which is crossed by the gradients of energy provided by the acoustic holographic interference field. The assembling of the particles may thereby take place in the aforementioned device, dispenser, and/or apparatus, e.g. in the fluid conduit, merging portion, and/or channel portion. As outlined in the context of the device, also by said method the particles can precisely be arranged to complex agglomerates, irrespective of the symmetry and geometry of the conduit the flow of the working medium is guided. Furthermore, the method does advantageously neither require any superposition of standing waves nor phase discontinuities to trap the particles in 3D.

According to an aspect, the particles may comprise at least one of the following components: a biological cell, a stem cell, a spheroid, an organoid, a tissue fragment, a biomolecule, a bacterium, a virus, a gel bead, a vesicle, a tissue scaffold material, a lipid drop, a hydrogel material, a macromolecule, a polymer, a ceramic particle, a metallic particle, a metal flake, a glass sphere, and a carbon fibre. Preferably, the same may also apply to the further particles and/or the other particles mentioned herein.

In addition or alternatively, the working medium may be hydrogel and/or water. Again, preferably, here the same may also apply for the further working medium and/or the other working medium.

According to another aspect, the step of emitting (the acoustic holographic interference field) may comprise emitting the acoustic holographic interference field in a pulsed manner. For example, the emitting may include a (e.g. periodic) on and off switching of the acoustic holographic interference field. Preferably, the emitting of the acoustic holographic interference field in the pulsed manner is for structuring an arrangement of particles along a flow direction of the flow of the working medium. For example, by generating the acoustic holographic interference field for a first period of time, an agglomerate may be formed within this period. By pausing the generation of the acoustic holographic interference field for a second period of time, the formed agglomerate is carried away by the flow of the working medium, while no further agglomeration takes place. By repeating this process, a (periodic) sequence of separated agglomerates may be formed along the flow direction of the working medium. Advantageously, this allows for transversally structuring the particles with respect to the flow of the working medium.

In addition or alternatively, the step of emitting may comprise generating the acoustic holographic interference field during an emission period (e.g. for assembling the particles to an agglomerate), and interrupting the generation of the acoustic holographic interference field during an idle period (e.g. for discharging the assembled agglomerate). Preferably, the aforementioned steps are repeated periodically. As mentioned before, this advantageously allows for creating a discontinuous structuring of the particles along the flow direction of the flow of the working medium.

According to another aspect, the method may further comprise merging (e.g. mixing) the flow of the working medium and a further flow of a further working medium to form a flow of a composite medium. For example, the flow of the working medium may be guided in a channel portion to a (common) merging portion, while the further flow of the further working medium may be guided to the (common) merging portion by a further channel portion. In this context, the flow of the working medium may also be referred to as a first flow of a first working medium, while the further flow of a further working medium may be referred to as a second flow of a second working medium. The first working medium and second working medium may preferably be different (e.g. different materials and/or concentrations). Alternatively, the first working medium and second working medium may also be of the same type. The second or further working medium may comprise further particles, which e.g. may be different from those of the first working medium. Alternatively, the second or further working medium may comprise no solid components and/or additives.

Further, the step of emitting (the acoustic holographic interference field) may comprise emitting the acoustic holographic interference field to the flow of the composite medium for manipulating the particles (and possibly also the further particles) in the flow of the composite medium. For example, the acoustic holographic interference field may be emitted to the common merging portion and/or a dispensing channel portion, which fluidically connects the merging portion to an outlet. Depending on the properties (e.g. compressibility, absorptivity, and/or reflectivity) of the particles, the further particles, the working medium, and/or the further working medium within the composite medium, advantageously, complex agglomerates may be formed (e.g. simultaneously) within a single processing step.

According to another aspect, the (e.g. first) working medium may differ from the further (e.g. second) working medium. For example, the (e.g. first) working medium and the further (e.g. second) working medium may consist of different materials (e.g. on may be a hydrogel including synthetic polymers, while the other may be a hydrogel including natural polymers) and/or different concentration/formulation.

In addition or alternatively, the further (e.g. second) working medium may comprise further (e.g. second) particles. The further particles may be organic (e.g. comprising biological cells, bacteria, organoids, tissue samples, hydrogels, gel beads, and/or polymers), inorganic (e.g. comprising glass, ceramics and/or metal), or any combination thereof. Preferably, the (e.g. first) particles and the further (e.g. second) particles respond differently to acoustic, electric and/or magnetic forces. In addition and or alternatively, the (e.g. first) particles and the further (e.g. second) particles may differ in size and/or material. In addition and or alternatively, the (e.g. first) particles and the further (e.g. second) particles each may include different biological cell lines. Preferably, one of the different biological cell lines may be larger than another of the different biological cell lines.

According to another aspect, the method may further comprise (e.g. metered) dispensing the working medium with the manipulated particles on a substrate (e.g. a sample carrier) by a dispenser. For example, the working medium with the manipulated particles may be dispensed on the substrate as a continuous fibre. Preferably, the step of dispensing comprises dispensing the working medium with the manipulated particles in a (e.g. two-dimensional) base layer. Particularly preferred, the step of dispensing may comprise dispensing a plurality of subsequent layers on the base layer for layerwise forming a three-dimensional object. Advantageously, this allows for an additive manufacturing of three-dimensional objects.

In addition or alternatively, the method may further comprise subjecting the dispensed working medium with the manipulated particles to a fixation (e.g. via a fixation device). The fixation may comprise a binding (e.g. a solidification and/or connection) of the particles, the working medium, and/or working medium constituents. Advantageously, various types of binding processes are available, which may be selected in dependency on the features of the particles and/or the working medium. If the particles include are, for example, reactive, i.e. capable of providing the binding reaction in their self, the fixation may be obtained simply by maintaining the dispensed working medium with the manipulated particles for a certain fixation time, e.g. in a range of seconds to hours. Alternatively, the fixation may be triggered by supplying a fixation input, like at least one of thermal energy input, an irradiation input and a fixation agent input. For applying the thermal energy input, the fixation device may include a thermal source, like e.g. an infrared radiation source, directing thermal energy to the assembling portion. Alternatively, a fixation agent, i.e. a chemical substance, which is capable of providing the binding process between the particles, may be supplied into the working medium. Consequently, the fixation may be preferably a result of biological growth, a timed reaction and/or caused by an external trigger, e.g. thermal energy, irradiation, and/or a fixation agent. Advantageously, the dispensed object can be preserved in its respective shape.

Further details and advantages of the disclosure are described in the following with reference to the attached drawings, which show in:
- Figures 1A-1D:: schematic illustrations of a device for manipulating particles in a flow of a working medium according to a first embodiment - mounted to a fluid conduit and standalone;
- Figures 2A-2D:: schematic illustrations of a device for manipulating particles in a flow of a working medium according to a second embodiment - mounted to a fluid conduit and standalone;
- Figures 3A-3C:: schematic illustrations of a device for manipulating particles in a flow of a working medium according to a third embodiment - mounted to a fluid conduit;
- Figures 4A-4C:: schematic illustrations of the manipulation of a working medium comprising two different types of particles reacting differently to acoustic forces by a device according to a fourth embodiment;
- Figure 4D:: sections of possible particle arrangements obtainable with a device for manipulating particles in a flow of a working medium according to an embodiment;
- Figures 5A-5D:: schematic illustrations of the dispensing of a working medium with manipulated particles by an apparatus according to an embodiment;
- Figures 6:: schematic illustration of a dispenser for dispensing a working medium with particles comprising a device according to an embodiment;
- Figures 7A-7B:: schematic illustrations of the dispensing of a working medium with manipulated particles by an apparatus according to another embodiment;
- Figures 8:: schematic illustration of a dispenser for dispensing a working medium with particles comprising a device according to an embodiment;
- Figures 9A-9C:: schematic illustrations of the manipulation of a working medium with particles and a further working medium with further particles by a dispenser according to an embodiment;
- Figures 10A-10C:: schematic illustration of a dispenser for dispensing a working medium with particles comprising a device according to an embodiment.

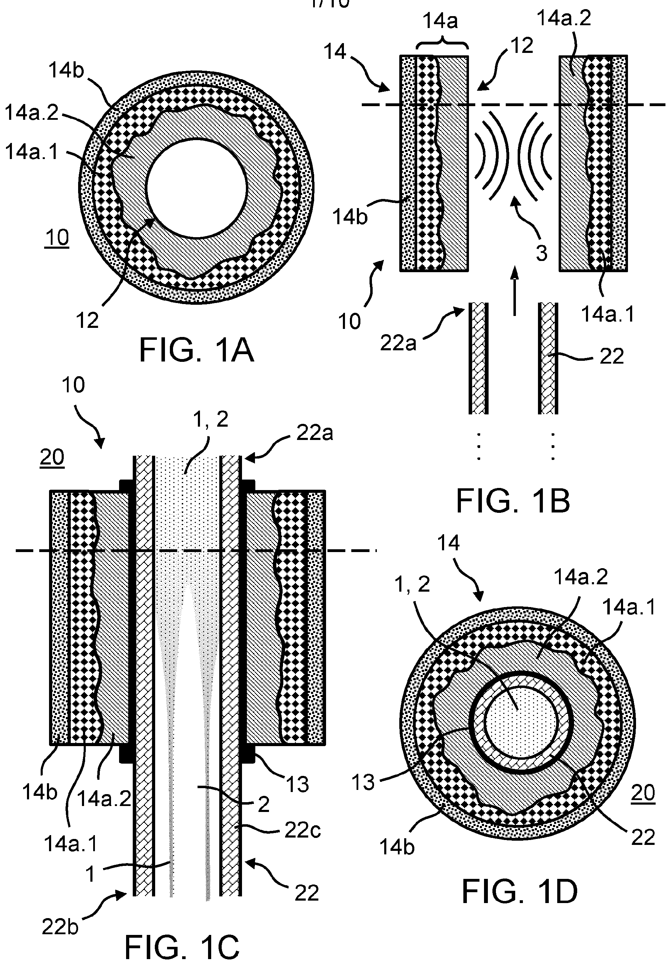

Figures 1A-1D, 2A-2D, 3A-3C, 4A-4C, 6, 8 and 10A-10C show schematic illustrations of devices 10 for manipulating particles 1 in a flow of a working medium 2 according to various embodiments. The illustrations comprise primarily side views of the device 10 and top views along sections indicated in the respective Figures by dashed lines. The device 10 may be standalone or form part of a dispenser 20 for dispensing the working medium 2 with the particles 1, and/or form part of an apparatus for fabricating structures of the working medium 2 with the particles 1. In the dispenser 20 and/or the apparatus, the device 10 may, for example, be mounted to a fluid conduit 22 of the dispenser 20 and/or the apparatus, wherein preferably the fluid conduit 22 may be for guiding the flow of the working medium 2 with the particles 1.

The particles 1 used in this context may be organic (e.g. comprising bacteria, tissue samples, hydrogels, and/or natural or synthetic polymers including, but not limited to, collagen, gelatin, silk, agarose, chitin, hyaluronan, alginate, dextran, cellulose, poly(caprolactone), poly(lactic acid), or polystyrene), inorganic (e.g. comprising glass, ceramics and/or metal), or any combination thereof. Further, the particles 1 may be contained (e.g. suspended) in a, preferably liquid, working medium 2 (e.g. water or a hydrogel). The particles 1 and the working medium 2 may, for example, form a suspension. In the following, the working medium 2 may also be referred to as printing medium. The working medium 2 comprising the particles 1 may also be referred to as ink or bioink.

The device 10 comprises a mounting portion 12 for mounting the device 10 to a fluid conduit 22 and an acoustic source component 14 connected to the mounting portion 12.

The mounting portion 12 and the acoustic source component 14 may be firmly attached to each other and/or integrally linked to each other. Preferably, the mounting portion 12 and the acoustic source component 14 cannot be separated from each other in a non-destructive way. For example, the mounting portion 12 is formed by a (e.g. cylindrical and/or annular) surface segment of the acoustic source component 14. The mounting portion 12 may e.g. be a recess in the acoustic source component 14. However, it is also possible that the mounting portion 12 and the acoustic source component 14 may be detachably attached to each other, e.g. that the components may be separated from each other (e.g. for maintenance).

The mounting portion 12 may be configured for accommodating the fluid conduit 22 and/or form an annular opening for inserting the fluid conduit 22 therein. The mounting portion 12 may be configured for being connected non-positively and/or positively with the fluid conduit 22. For example, the mounting portion 12 may have an inner contour, which is adapted to fit to an outer contour of the fluid conduit 22. The mounting portion 12 and/or the device 10 may be configured for being pushed onto and/or pressed onto the fluid conduit 22. For sealing a joint between the fluid conduit 22 and the mounting portion 12 may optionally comprise a sealing element (e.g. a deformable pad or film, not shown). In an embodiment, the mounting portion 12 and/or the device 10 may be segmented and/or comprising several segments (e.g. two half-shells). The segments may be, for example, configured for being joined together around the fluid conduit 22.

The fluid conduit 22 may be, for example, a fluid pipe and/or fluid tube (e.g. such as from a syringe needle, nozzle and/or a pipette). The fluid conduit 22 may be for guiding the flow of the working medium 2. The fluid conduit 22 may be of stainless steel and/or plastic. The fluid conduit 22 may comprise a (e.g. first) end portion 22a, a further (e.g. second) end portion 22b, and a (e.g. closed) connection portion 22c fluidically connecting the respective end portions 22a, 22b. Preferably, the connection portion 22c has a constant cross-section. However, the connection portion 22c may also be tapered.

The acoustic source component 14 may be configured for emitting an acoustic holographic interference field 3 manipulating the particles 1 in the flow of the working medium 2. Preferably, the acoustic holographic interference field 3 defines a shape of an agglomerate of particles to be formed. The acoustic holographic interference field 3 may, therefore, have a configuration/pattern, which may be (set) such that it corresponds to that of the agglomerate to be assembled, which will be outlined in more detail herein. The acoustic source component 14 may be configured for emitting acoustic waves with frequencies in a non-audible frequency range, in particular ultrasound frequencies. Preferably, the acoustic source component 14 is frequency-tunable. For example, the acoustic source component 14 may be configured for emitting sound waves in a frequency range from 100 kHz to 50 MHz. Consequently, the acoustic holographic interference field 3 may have an acoustic holographic interference field frequency in the ultrasound frequency range, e.g. in a frequency range from 100 kHz to 50 MHz. The acoustic source component 14 may comprise at least one ultrasonic transducer 14b (e.g. at least one piezoelectric ultrasonic transducer). In an embodiment, the acoustic source component 14 and/or the at least one ultrasonic transducer 14b may comprise only one single ultrasonic transducer. However, alternatively, the acoustic source component 14 and/or the at least one ultrasonic transducer 14b may also comprise several (e.g. two, three, four, or six) ultrasonic transducers 14b. The several ultrasonic transducers may be configured identical and/or configured for (e.g. synchronously) emitting acoustic waves of the same frequency. The at least one ultrasonic transducer 14b may have an operating frequency in the range of 0.1 MHz to 100 MHz. The at least one ultrasonic transducer 14b may provide high acoustic power output (> 1 W) over long time scales (continuous wave operation). The at least one ultrasonic transducer 14b may be water-cooled or air-cooled for optimal performance. The at least one ultrasonic transducer 14b may be operated in a pulsed and/or sequential operation.

As shown in Figure 1A-1D, the at least one ultrasonic transducer 14b may comprise a cylindrical and/or tubular ultrasonic transducer (e.g. a piezo tube actuator). In this context, the cylindrical and/or tubular ultrasonic transducer may comprise an active element for generating mechanical vibrations, which has a tubular, hollow-cylindrical and/or any other hollow-like shape. The cylindrical and/or tubular ultrasonic transducer may be configured for emitting sound waves radially inwards, e.g. to a centre of a central passage running through the cylindrical and/or tubular ultrasonic transducer.

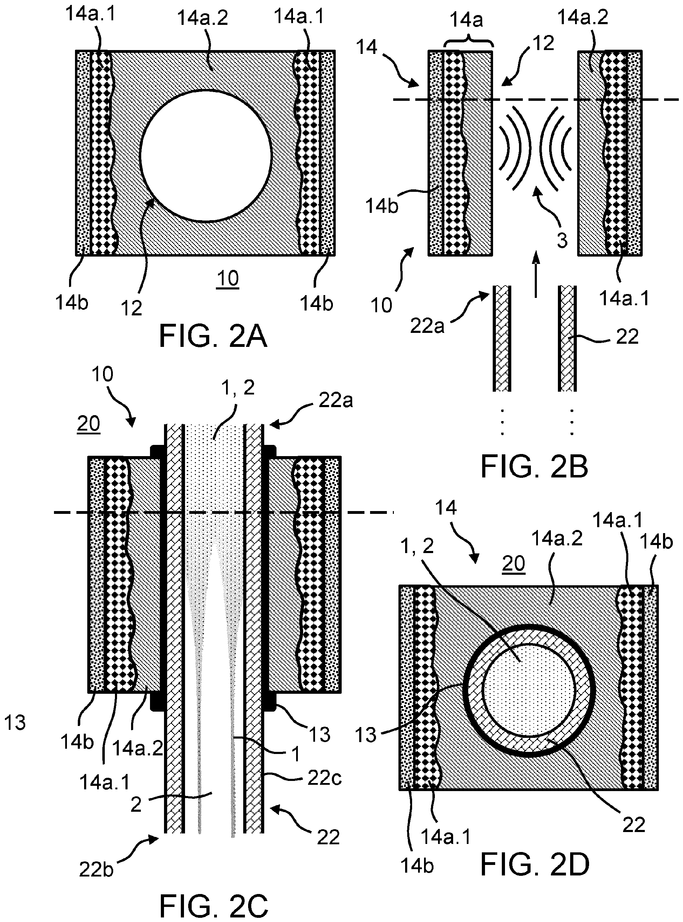

In addition or alternatively, as shown in Figure 2A-2D, the at least one ultrasonic transducer 14b may comprise an ultrasonic plate transducer (e.g. a piezo plate actuator). The ultrasonic plate transducer may be flat and/or non-curved. The ultrasonic plate transducer may have a planar (e.g. circular, square or rectangular) active element for generating mechanical vibrations. The ultrasonic plate transducer may be configured for emitting sound waves perpendicular to a plate plane. Preferably, the at least one ultrasonic transducer 14b may comprise at least two or more ultrasonic plate transducers. These may, for example, be arranged parallel to each other and/or facing each other, as shown in Figure 1A and 1D. The at least two or more ultrasonic plate transducers may, however, also be arranged tilted to each other (e.g. with an angle of 90° to each other). Preferably, the at least two or more ultrasonic plate transducers may be arranged concentrically around a common centre (e.g. the assembling portion). For example, the at least one ultrasonic transducer 14b may comprise a (e.g. annular and/or circular) phased array transducer, e.g. comprising twelve (e.g. individually controllable) transducers arranged in a ring.

For generating/emitting the acoustic holographic interference field 3, the acoustic source component 14 may comprise an acoustic diffractive element 14a. The acoustic diffractive element 14a may be a (e.g. static) acoustic hologram (e.g. transmission hologram), a (e.g. dynamic) spatial acoustic modulator (e.g. a microbubble array), and/or an acoustic (e.g. labyrinthine) metamaterial. The acoustic diffractive element 14a may be placed on a face or emitting surface of the at least one ultrasonic transducer 14b. Preferably, the acoustic source component 14 only comprises a single or common acoustic diffractive element 14a, to which each of the at least one ultrasonic transducer 14b is connected and/or coupled. For example, in case the at least one ultrasonic transducer 14b comprises several ultrasonic transducers 14b, these may be arranged (e.g. distributed) at an outer surface of the acoustic diffractive element 14a. The acoustic diffractive element 14a may be fixedly connected to the at least one ultrasonic transducer 14b. Preferably, the acoustic diffractive element 14a is, however, removable and/or replaceable. Thus, by exchanging the acoustic diffractive element 14a, different structures and/or agglomerates may be assembled with the device 10. The acoustic diffractive element 14a may shape the sound waves emitted by the at least one ultrasonic transducer 14b in a way that they form the acoustic holographic interference field 3 in a shape of an agglomerate to be created. In the acoustic holographic interference field 3, the particles 1 may rearrange and assemble due to acoustic radiation forces and form the agglomerate.

As shown, for example, in Figure 1A-1D, the acoustic diffractive element 14a and/or the acoustic hologram may comprise at least two diffractive layers 14a.1 and 14a.2, e.g. an outer diffractive layer 14a.1 and an inner diffractive layer 14a.2. The at least two diffractive layers 14a.1, 14a.2 may have different acoustic properties. For example, the propagation of sound may differ within the at least two diffractive layers 14a.1, 14a.2. The at least two diffractive layers 14a.1, 14a.2 may be (e.g. fixedly) connected and/or coupled to each other. For example, the at least two diffractive layers 14a.1, 14a.2 may be arranged concentrically and/or coaxially, e.g. around a common centre (e.g. the assembling portion). The acoustic diffractive element 14a and/or the acoustic hologram may further comprise at least one (e.g. predetermined) interface, where two of the at least two diffractive layers meet and/or abut each other. A shape of this at least one interface and/or the material properties of the at least two diffractive layers 14a.1, 14a.2 may determine the acoustic holographic interference field 3, e.g. how the primary acoustic wave is shaped. For example, the structure of the acoustic hologram and in particular the structure of the at least one interface may be configured and/or manufactured such that a specific acoustic holographic interference field is generated, resulting in an agglomeration of the particles 1 in a specific shape and/size. This may be done, for example, using an algorithm for computer-based calculation based on the Gerchberg-Saxton-Algorithm.

The acoustic diffractive element 14a and/or the acoustic hologram may have a tubular, hollow-cylindrical and/or any other hollow-like shape. The diffractive element 14a and/or the acoustic hologram may comprise a (e.g. central) through hole. For example, the through hole may be formed and/or delimited by an inner surface of the acoustic diffractive element 14a and/or the acoustic hologram, e.g. an inner surface of the inner diffractive layer 14a.2. This through hole may, for example, have a constant cross-section. Alternatively, the through hole may also have a varying (e.g. tapered) cross-section. The through hole and/or the inner surface of the acoustic diffractive element 14a (e.g. the inner surface of the inner diffractive layer 14a.2) may form and/or delimit (at least partly) the mounting portion 12. Consequently, the acoustic diffractive element 14a and/or the acoustic hologram may (e.g. circumferentially and/or concentrically) surround the mounting portion 12.

Figures 1C and 2C, for example, show configurations, where the device 10 is mounted to the fluid conduit 22. In this embodiments, the device 10 is mounted onto fluid conduit 22, e.g. in a belt-like or sleeve-like manner. For example, the device 10 is mounted onto the connecting portion 22c of the fluid conduit 22. Consequently, the fluid conduit 22 completely may pass through the device 10. In this context, it should be mentioned that the fluid conduit 22 does not necessarily have to be straight, as shown in the respective figures. The fluid conduit 22 may also be conical, such as the shape of a pipette tip. In such a case, the mounting portion 12 and/or the acoustic source component 14 may preferably be correspondingly (e.g. conical) shaped, e.g. for providing a conical through hole of same dimensions. This may provide a tight fit between the device 10 and the fluid conduit 22. As also shown in 1C, 1D, 2C and 2D, the arrangement or dispenser 20 may also comprise a (e.g. fluid) acoustic coupling layer 13 arranged between the mounting portion 12 and the fluid conduit 22, preferably for providing effective sound transmission between the fluid conduit 22 and the device 10. The acoustic coupling layer 13 may be, for example, water, glycerol, and/or ethylene glycol.

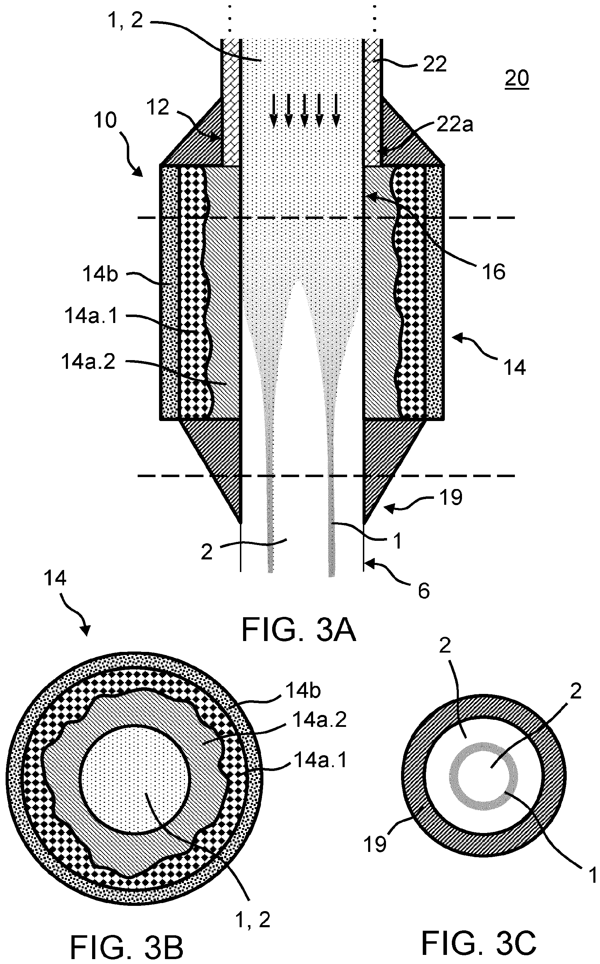

Alternatively, as shown for example in Figure 3A, it is also possible that (only) the end portion 22a (e.g. the first end portion) of the fluid conduit 22 is mounted to the mounting portion 12 and/or the device 10, e.g. when the mounting portion 12 forms part of an inlet of the device 10. In this context, the device 10 may further comprise a (e.g. straight) channel portion 16 for receiving and guiding the flow of the working medium 2. The channel portion 16 may be fluidically connectable or connected to the fluid conduit 22. For example, the mounting portion 12 may be arranged at an end of the channel portion 16, such that when the fluid conduit 22 is mounted to the mounting portion 12, a fluid connection between the channel portion 16 and the fluid conduit 22 is formed. The channel portion 16 may be arranged downstream to the fluid conduit 22 and/or the mounting portion 12. The channel portion 16 may be pipe-, tube, and/or hose-like. Preferably, the channel portion 16 may be (e.g. coaxially) surrounded by the acoustic source component 14 and/or the acoustic diffractive element 14a. For example, the through hole and/or the inner surface of the acoustic diffractive element 14a (e.g. the inner surface of the inner diffractive layer 14a.2) may form and/or delimit (at least partly) the channel portion 16.

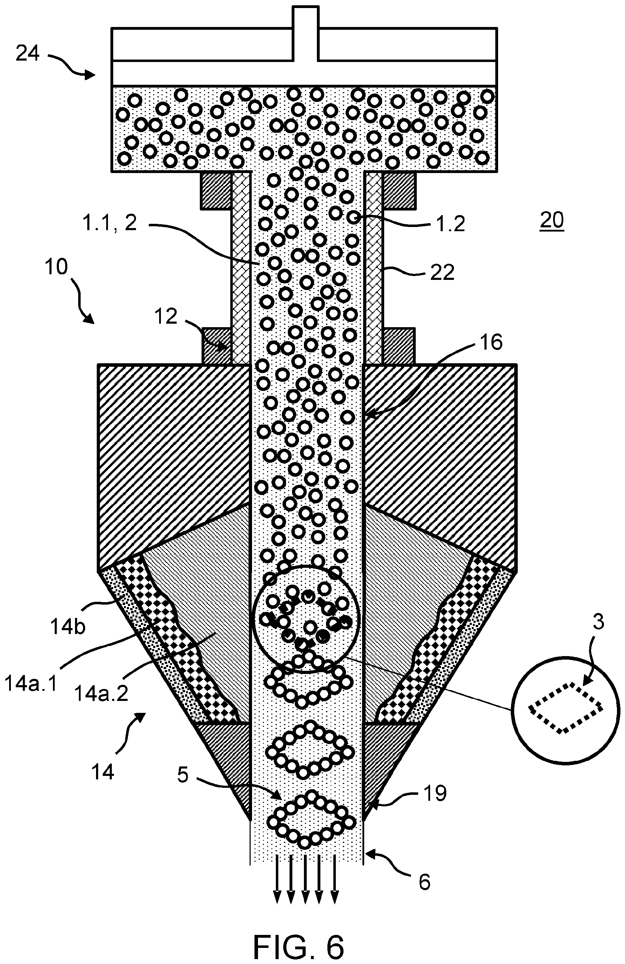

Before further structural details of the device 10 are discussed in more detail below, the manipulation of the particles 1 is described. As illustrated by the arrows in the figures a, preferably constant, flow of the working medium 2 (e.g. a hydrogel precursor) with the particles 1 (e.g. cells) is provided. For example, the working medium 2 with the particles 1 may be pushed through the fluid conduit 22, e.g. by an extruder component 24 (see e.g. Figure 6). A flow speed of the flow of the working medium 2 (e.g. the extrusion speed) may be set by the pressure applied by the extruder device 24. Further, an acoustic holographic interference field 3 is emitted by the acoustic source component 14 to the flow of the working medium 2, e.g. to the centre of the mounting portion 12 and/or the centre of the channel portion 16. When the particles 1 feel the acoustic holographic interference field 3, the particles move relative to the flow direction under the effect of acoustic forces. Depending on the acoustic holographic interference field 3, the particles may agglomerate and/or concentrate, wherein a resulting shape depends on the projected acoustic holographic interference field 3. By varying an input power of the acoustic source component 14, an amplitude of the acoustic holographic interference field 3 and the resulting acoustic radiation forces can be controlled. An amplitude of the acoustic radiation forces in turn determines a speed at which particles 1 rearrange and can be used to create density gradients in the final product. Due to the flow (or positive liquid displacement) of the working medium 2, the assembled and/or manipulated particles 1 may be further transported and the working medium 2 with the assembled particles 1 may be extruded as a continuous filament 6, e.g. out of an (e.g. nozzle-like) outlet 19 of the dispenser 20.

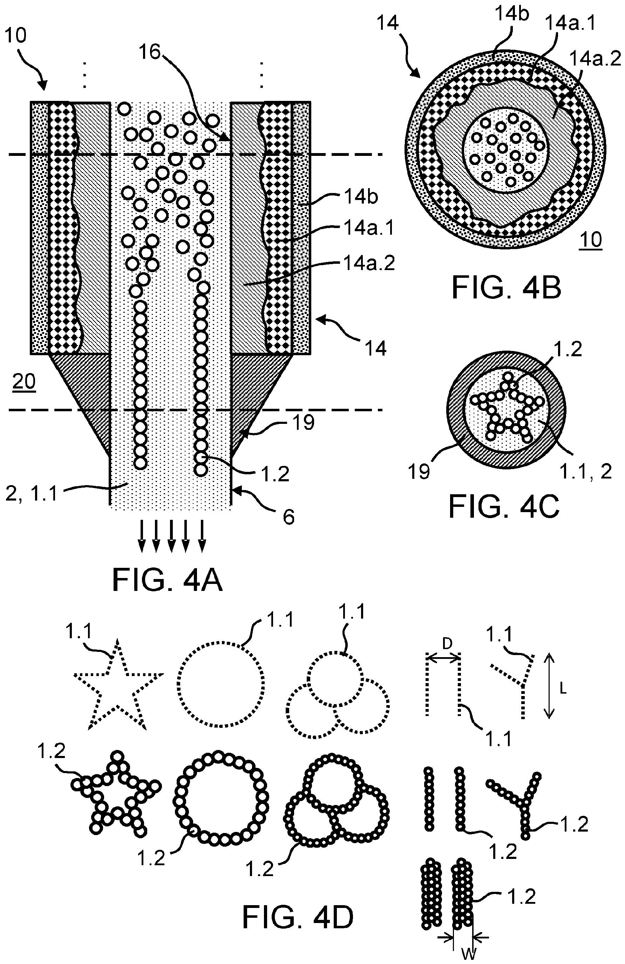

In this context, as shown, for example, in Figures 4A-4C, the working medium 2 or ink may comprise two different types of particles 1, e.g. two different types of cells. For example, the working medium and/or particles may comprise a first type of particles 1.1 (e.g. a first type of cells) and a second type of particles 1.2 (e.g. a second type of cells), wherein the second type of particles 1.2 is different from the first type of particles 1.1. The first type of particles 1.1 may be indifferent to the acoustic holographic interference field emitted by the acoustic source component 14. This may be due to a small particle size or a material composition of the first type of particles 1.1. For example, the first type of particles 1.1 may have the same density as the working medium 2. The second type of particles 1.2 may react to the acoustic holographic interference field 3 and thus may be acoustically manipulated via the acoustic holographic interference field 3. For example, the size or material composition of the second type of particles 1.2 may be chosen appropriately. The second type of particles 1.2 may e.g. be spheroids, organoids, aggregates of biological cells, aggregates of biological cells and inorganic matter, and/or hydrogel bead or polymer bead containing one or more of the aforementioned materials. Figures 4B and 4C show cross-sections along the dashed lines in Figure 4A, showing the arrangement of the particles 1, i.e. the first and second type of particles 1.1 and 1.2., before and after interacting with the acoustic holographic interference field 3. Figure 4C thereby illustrates the fact that with the claimed device 10 it is also possible to create shapes/symmetries, which are independent of the shape of the fluid conduit 22 and/or channel portion 16.

Figure 4D shows cross-sections of possible particle arrangements, which may be obtained via acoustic manipulation described herein. The particle arrangements may include particle arrangements of a first type of particles 1.1 and/or a second type of particles 1.2, wherein the first and second type of particles 1.1, 1.2 may differ in particle size. The particle arrangements may have a cross-sectional shapes which consists of only one single line of particles. Alternatively, the line may also be multiple particles wide. The crossectional shapes of the assembled particle agglomerates are also not limited to closed forms, like rings, stars or ovals. Further, the shapes may not be connected. That means, the particles do not need to be in contact and the target pattern can be chosen so that there are fixed distances between subshapes (e.g. see distance "D" separating the parallel lines in Figure 4D). The shape may be set e.g. by calculating and fabricating a respective acoustic hologram or acoustic diffractive element 14a, which may also set a characteristic lengths (e.g. maximum extent "L") of a shape.

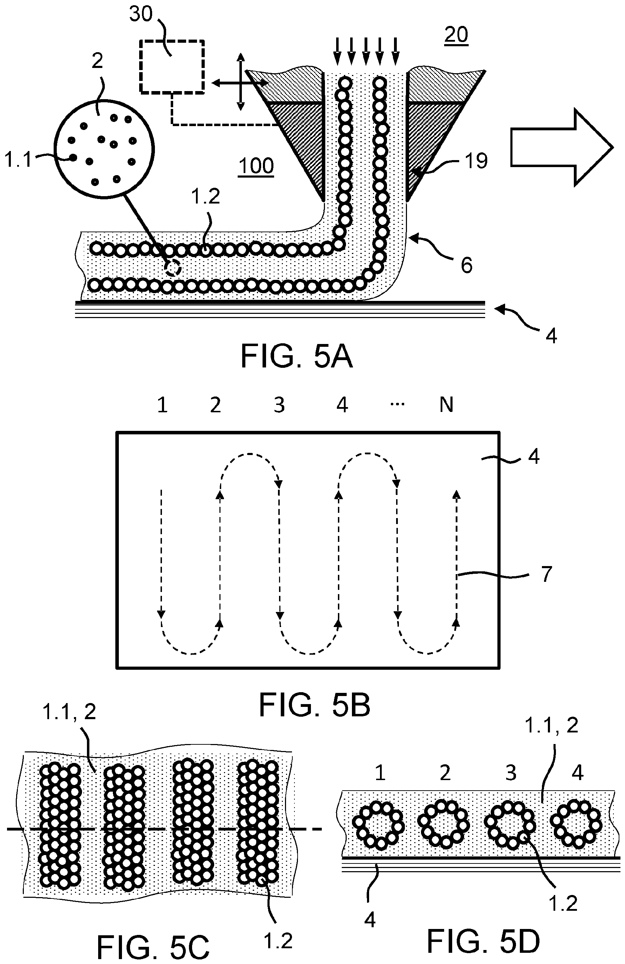

Figures 5A-5D relate to dispensing a filament 6 of working medium 2 with the manipulated particles 1 on a substrate 4, e.g. via an apparatus 100. The apparatus 100 may comprise dispenser 20 with a device 10 and a positioning device 30 configured for changing a relative position of the dispenser 20 to the substrate 4, preferably in three spatial directions. Exemplarily, the working medium 2 again comprises two different types of particles 1, namely a first type of particles 1.1 and a second type of particles 1.2 reacting differently to the applied acoustic holographic interference field 3. Exemplarily, the filaments 6 may comprise tissue strands, which consist of the second type of particles 1.2. These second type of particles 1.2 were acoustically assembled to form connected tissue strands. The properties of the ink may be chosen to prevent internal fluid flows inside the filament 6 to prevent mixing and disintegration of the tissue strand's shape. The ink further contains the first type of particles 1.1, which did not experience acoustic radiation force in the acoustic holographic interference field 3 and thus are still homogeneously distributed through the volume of the filament 6 (see inset Figure 5A). Besides dispensing the working medium 2 with the manipulated particles 1 on a substrate 4, the working medium 2 with the manipulated particles 1 may, alternatively, also be dispensed on a previously deposited layer (e.g. a base layer), e.g. during additive manufacturing. The working medium 2 with the manipulated particles 1 may, thereby, be extruded out of the dispenser 20 in a liquid or gel-like state and may then be subjected to a fixation, such that the working medium 2 solidifies within a certain time. The certain time may have to be chosen according to process parameters such as viscosity of the ink and/or filament 6 diameter to prevent disintegration of the shape of ink filament 6 and particle assembly. The fixation (e.g. gelling and/or solidification) process may be initiated via thermal, chemical, mechanical, electromagnetic or optical elements. During the dispensing process the dispenser 20, the substrate 4, or both may be moved, e.g. by a positioning device (not shown), resulting in a relative motion to each other. One example of a printing trajectory 7 is shown in Figure 5B as a meandering path. Figure 5C shows an illustrative top view of parts of the dispensed structures, while Figure 5D shows a side view section along the dashed line in Figure 5C. As can be seen in Figure 5A, the second type of particles 1.2 are arranged in a strand of tubular shape. This results in similar tubular structures on the substrate, which are aligned with the printing trajectory 7 (see Figure 5C and 5D). Notably, the dimensions (e.g. inside diameter of tubular filaments) can be much smaller than an opening diameter of the outlet 16 of the dispenser 20.

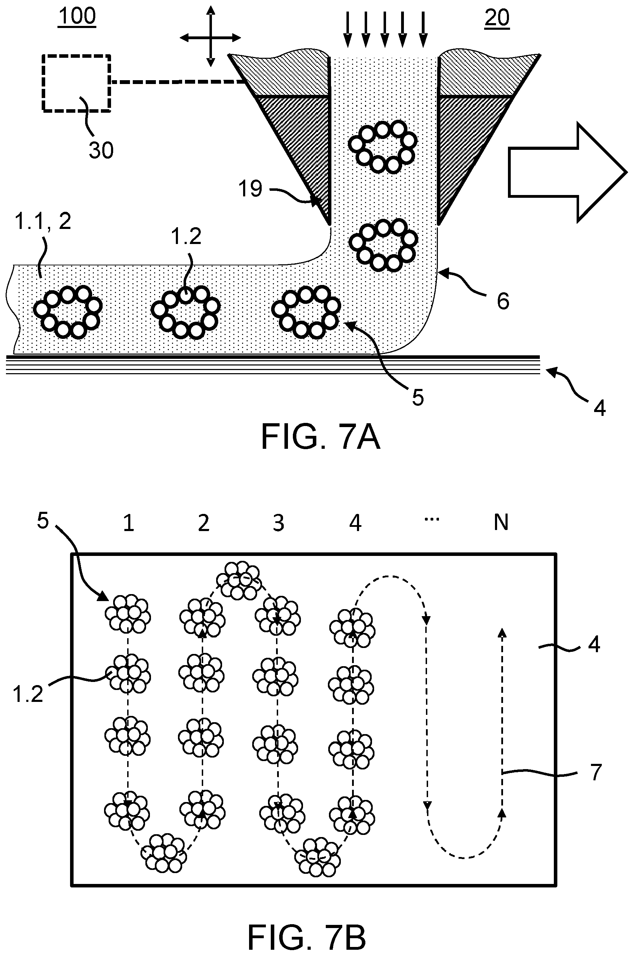

In addition or alternatively to the aforementioned assembling of continuous particle structures, like tubes and/or strands, also finite structures or shaped agglomerates 5 may be formed during the continuous flow of ink, as illustrated in Figure 6. For this, the acoustic holographic interference field 3 has to be pulsed, e.g. with a certain pulse duration. In other words, the acoustic holographic interference field 3 may be emitted in a pulsed manner. A time for the acoustic holographic interference field 3 to be fully formed may thereby depend on the distance between the acoustic source component 14 and the assembling portion. This time and the relative flow speed (continuous flow) may determine the minimum separation distance between two agglomerates 5. The actual separation distance may be set by duration of an ON-state or emission period, wherein the acoustic holographic interference field 3 is generated. During the ON-state the particles 1, the second type of particles 1.2, may be trapped and assemble (in the assembling portion) under the effect of acoustic forces. Depending on the initial concentration of particles 1 in the inks the agglomerate 5 may grow at a certain rate. There may be a maximum size of the agglomerate 5, depending on if all the acoustic traps are filled with particles 1 (e.g. with the second type of particles 1.2). Beyond this maximum size, no more particles 1 can be trapped in the agglomerate 5 and will thus be discharged by the flow. This may lead to an upper limit for the time duration of the ON-state, depending on the pattern to be created, the flow speed and the particle concentration in the ink. Then the generation of the acoustic holographic interference field 3 may be interrupted for an idle period or OFF-state. During the OFF-state no acoustic holographic interference field 3 may be generated, thus no particles 1 may be trapped and assemble during this time. An exemplary resulting structured arrangement of particles along a flow direction of the flow of the working medium is shown in Figure 7A and 7B. Here, a continuous filament 6 of ink with agglomerates 5 at a certain distance to each other is dispensed on a substrate 4. As can be seen in Figure 7B, the final arrangement of the agglomerates 5 on the substrate 4 depends on the printing trajectory 7.

For assembling finite (3D) agglomerates 5 in the flow of the working medium 2, it is advantageous (but not necessary) to emit the acoustic holographic interference field 3 at an angle counter to the flow direction (see arrows). As exemplarily shown in Figure 6, the acoustic source component 14 may thus be arranged for emitting the acoustic holographic interference field 3 tilted with respect to a flow direction of the flow of the working medium 2. For example, the at least one ultrasonic transducer 14b may comprise a conical and/or frustum-like ultrasonic transducer (e.g. a truncated conical shell with a piezoelectric layer). The conical and/or frustum-like ultrasonic transducer may be configured for emitting sound waves at least partially axially, preferably counter-parallel, to the flow of the working medium 2. This may be to provide higher acoustic radiation forces against the drag of the flowing ink.

Though using an acoustic holographic interference field 3 orientated at least in parts counter-parallel to the flow, the assembled agglomerates 5 may be distorted in shape while travelling downstream the acoustic source component 14. This may be because in the regime of typical fluid duct dimensions in printheads (e.g. in the order 1 mm or more commonly less than 1 mm) the flow may be viscosity dominated. This regime may be called Poiseuille flow and characterized by a parabolic variation of the flow speed across the channel portion. In the centre of the channel portion, the liquid and also the particles 1 may travel faster than those which are located closer to the channel portion walls. Once the ink is outside the channel portion, this may be no longer the case and no more distortion may occur, at least due to this phenomenon. The distortion may be compensated by projecting a modified target pattern. Since the Poiseuille flow is reversible, a virtual agglomerate may be placed at the outlet 19 and the particle's parabolic flow profile may be computed back to the assembling portion. The result of the simulation may be taken into account in the generation of the acoustic holographic interference field 3.

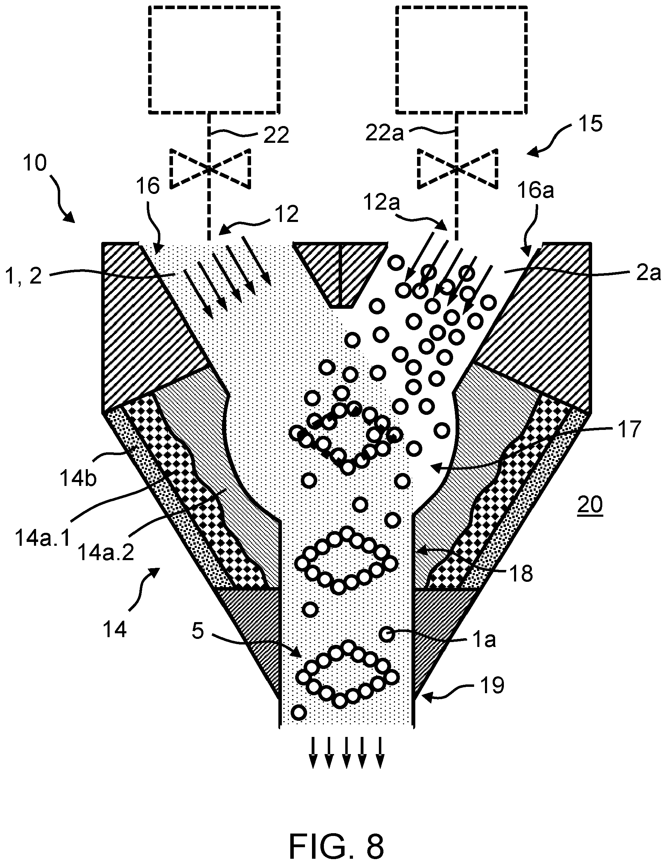

As can be seen in Figure 8, the device 10 may also comprise a further (e.g. second) channel portion 16a configured for receiving and guiding a further (e.g. second) flow of a further (e.g. second) working medium 2a. Consequently, the device 10 may, for example, comprise a first channel portion 16 configured for receiving and guiding a first flow of a first working medium 2 with first particles 1 and a second channel portion 16b configured for receiving and guiding a second flow of a second working medium 2a with second particles 1a. To supply the second channel portion 16a with the second flow of the second working medium 2a, the device 10 may further comprise a further (e.g. second) mounting portion 12a for mounting the device 10 to a further (e.g. second) fluid conduit 22a. Thereby, the further or second channel portion 16a may be fluidically connectable or connected to the further fluid conduit 22a. The first working medium 2 and second working medium 2a may be of different materials (e.g. one may be a hydrogel including synthetic polymers, while the other may be a hydrogel including natural polymers) and/or different concentration/formulation. In addition or alternatively, the first particles 1 and the second particles 1a may be different (e.g. two different types of cells). For example, the first particles 1 and the second particles 1a may respond differently to acoustic, electric and/or magnetic forces; and/or differ in size and/or material.

The device 10 may further comprise a merging portion 17, where the second channel portion 16a joins the first channel portion 16. The first working medium 2 and second working medium 2a, i.e. the two inks, may be brought together in the merging portion 17. In the merging portion 17, the first and second channel portion 16, 16a may merge, e.g. for becoming one channel (e.g. a dispensing channel portion 18 fluidically connecting the merging portion 17 to an outlet 19 of the device 10). Consequently, this setup may be used to merge the flow of the working medium 2 and the further flow of the further working medium 2a to form a flow of a composite medium. For example, the setup may be used to provide different ink formulations together and change the composition of the resulting composite medium during the flow (e.g. to print concentration gradients or density gradients). In this context, the device 10 may further comprise a fluid control component 15 configured to selectively allow or disallow a fluid flow in the channel portion 16 and the further channel portion 16a and/or to selectively increase or decrease a fluid flow rate in the channel portion and the further channel portion. For example, the fluid control component 15 may comprise fluidic switches (e.g. switching valves), wherein each fluidic switch is associated with one of the fluid conduit 22 and the further fluid conduit 22a, preferably for independently blocking or enabling the fluid flow in the fluid conduit 22 and the further fluid conduit 22a. In addition or alternatively, the fluid control component 15 may comprise fluidic regulators (e.g. control valves and/or flow regulating valves), wherein each fluidic regulator is associated with one of the fluid conduit 22 and the further fluid conduit 22a, preferably for independently regulating a fluid flow rate in the fluid conduit 22 and the further fluid conduit 22a.

The acoustic holographic interference field 3 may be projected to the merging portion 17 to assemble the particles (e.g. cells) of the composite medium to an agglomerate 5. In other words, the acoustic holographic interference 3 field may be emitted to the flow of the composite medium for manipulating the particles 1 in the flow of the composite medium. For this, the acoustic source component 14 may be configured and/or arranged to (e.g. circumferentially) surround the merging portion 17 and/or to focus the acoustic holographic interference field 3 into the merging portion 17. Also here, the shape of the agglomerate 5 is advantageously independent of the overall geometry of the merging portion 17. In another embodiment, the merging portion 17 may be used to focus the acoustic holographic interference 3 field into a smaller volume similar to a focusing lens. As can be seen in Fig. 8, a boundary between the inner diffractive layer 14a.2 and the merging portion 17 may be of concave shape. Considering a higher sound speed in the inner diffractive layer 14a.2 than in the working medium 2, such a concave shape may result in a focusing of the acoustic holographic interference field 3. In this case, the interface between the inner diffractive layer 14a.2 and the merging portion 17 may work as a lens that demagnifies the acoustic holographic interference field 3 into a smaller working region.

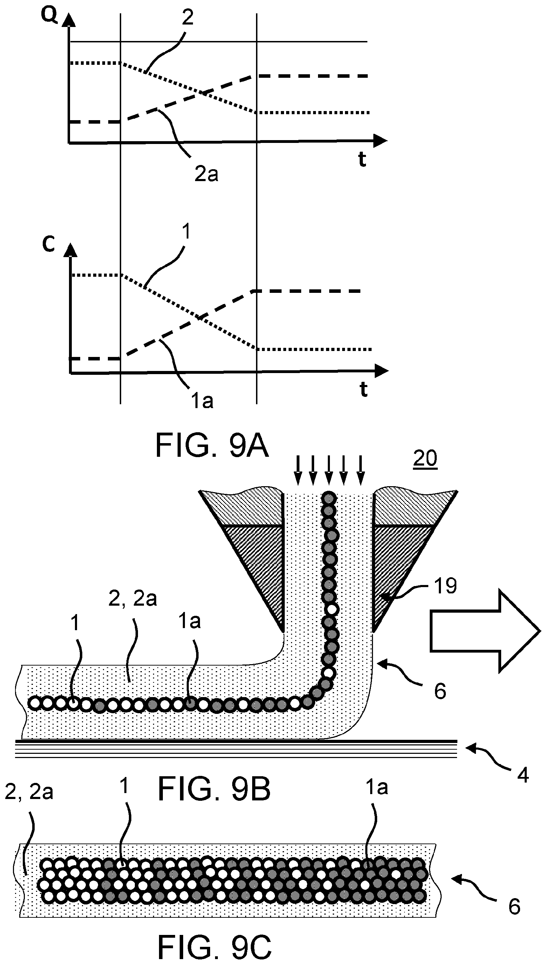

As shown in Figure 9A, a flow rate (flow rate Q in volume/time) of a first flow of a first working medium 2 (e.g. guided in the first channel portion 16) versus a flow rate of a second flow of a second working medium 2a (e.g. guided in the second channel portion 16a) may be changed. Generally, a speed (e.g. print speed) of moving a dispenser 20 or printhead relative to a substrate 4 may have to be balanced with the flow rate of the used bioink out of dispenser 20 or printhead. If the total flow rate (Q. = Q₁ + Q₂) remains constant, the speed may stay constant. If the flow rate were increased, the speed should be increased to provide similar results. The concentration of particles in a printing medium or working medium may be defined as number per volume. Therefore, the concentration of a first particles 1 in a printed object may be C₁ = (C₁ᵢₙ^{∗}Q₁)/(Q₁+Q₂) and similarly the concentration of a second particles 1a may be C₂ = (C₂ᵢₙ∗Q₂)/(Q₁+Q₂). There Q₁, Q₂ may be the flow rates of the first flow of the first working 2 and the second flow of the second working 2a, respectively. C₁ᵢₙ, C₂ᵢₙ may be the concentrations of first particles 1 within the first flow of the first working 2 and second particles 1a within the second flow of the second working 2a, respectively. If the flow rate of the first flow, for example, decreases and the flow rate of the second flow increases, the total flow rate may remain constant. Consequently, the concentration of the first particles 1 may decrease, while the concentration of the second particle 1a may increase (see Figure 8A bottom).

With this in mind, Figure 9B and 9C displays an exemplary output for a flat tissue strand of particles with varying concentrations of said particles (Figure 8B side view; Figure 8C top view). In a direction, the dispenser 20 is moving (see arrow), the concentration of the first particles 1 (shown in white) decreases. From this, it is further evident that the different types of particles may all be aligned to the same plane on the substrate even though the particles were feed to the dispenser 20 via different inlets. Such an arrangement would not be possible with a standing wave arrangement known in the prior art.

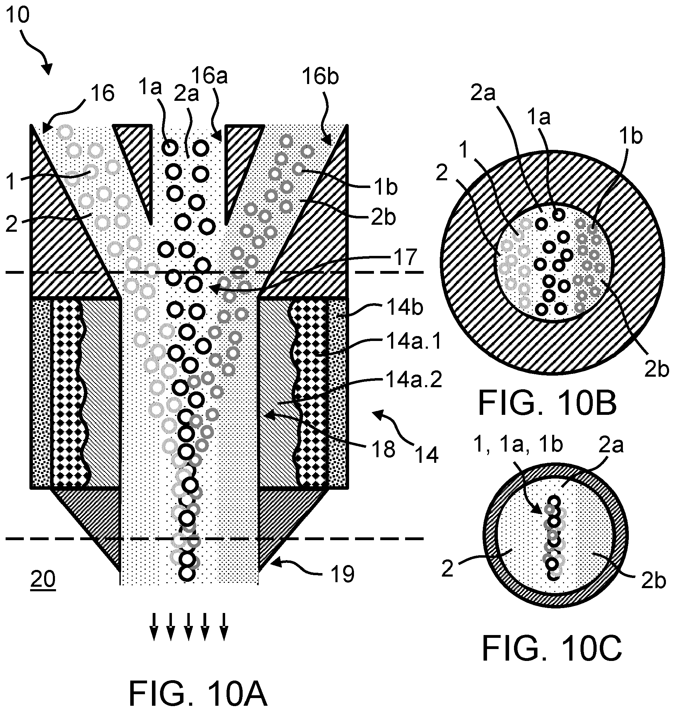

As shown in Figure 10A-10C, the device 10 may further comprise another (e.g. third) channel portion 16b configured for receiving and guiding another (e.g. third) flow of another (e.g. third) working medium 2b. Consequently, the device 10 may, for example, comprise a first channel portion 16 configured for receiving and guiding a first flow of a first working medium 2 with first particles 1, a second channel portion 16a configured for receiving and guiding a second flow of a second working medium 2a with second particles 1a, and a third channel portion 16b configured for receiving and guiding a third flow of a second working medium 2b with third particles 1b. In this context, it is evident, that the number of channel portions is not limited, such that the device 10 may also comprise additional (e.g. a fourth, fifth, ...) channel portions. The first, second, and third working medium 2, 2a, 2b may each be of different materials and/or different concentration/formulation. In addition or alternatively, the first, second and third particles 1, 1a, 1b may each be different. The first, second, and third channel portion 16, 16a, 16b may all join in the (common) merging portion 17. Thus, the flow of the first working medium 2, the flow of the second working medium 2a, and the flow of the third working medium 2b may be merged (e.g. in the merging portion) to form a flow of a composite medium. Alternatively, to focussing the acoustic holographic interference field 3 into the merging portion 17 (see Figure 8), the acoustic holographic interference field 3 may also be focussed and/or projected to a dispensing channel portion 18 configured for receiving and guiding the flow of the composite medium. For example, the different channel portions may be combined into one single channel portion (e.g. the dispensing channel portion 18) before entering the acoustic source component 14. The dispensing channel portion 18 may e.g. fluidically connect the merging portion 17 to an outlet 19 of the device 10. In this context, the acoustic source component 14 may be configured to (e.g. circumferentially) surround, the dispensing channel portion 18 and/or to focus the acoustic holographic interference field 3 into the dispensing channel portion 18.

In an embodiment, the different channel portions may have dimensions of less than 2 mm, preferably less than 1 mm diameter. Here, the different flows may be viscosity dominated (low Reynolds number) and consequentially two or more working media may not readily mix into each other. Instead, mixing may be limited by diffusion of the working media molecules. Thus, without agitation, the different working media or inks may run in parallel in the merging portion 17 and/or dispensing channel portion 18. However, the respective particles may experience (e.g. different) acoustic radiation forces in the acoustic holographic interference field 3. These acoustic radiation forces may transport the respective particles to the assembling portion without directly affecting the individual working media, which may mostly only mix due to diffusion (and some due to the entrainment of moving particles). Due to the acoustic radiation forces, the respective particles may be aggregated into overlaying patterns. This can be used to print varying concentration gradients of particles.

Although the invention has been described with reference to certain exemplary embodiments, it is evident to a person skilled in the art, that various changes can be implemented and equivalents can be used as substitute without departing from the scope of the invention. Consequently, the invention is not to be limited to the disclosed exemplary embodiments, but is to comprise all exemplary embodiments falling within the scope of the attached patent claims. More particularly, the invention also claims protection for the subject-matter and the features of the dependent claims independently of the referenced claims.

### Reference list

- 1: particle
- 1a: further particles
- 1b: other particles
- 2: working medium
- 2a: further working medium
- 2b: other working medium
- 3: first acoustic holographic interference field
- 4: substrate
- 5: agglomerate
- 6: filament
- 7: printing trajectory
- 10: device
- 12: mounting portion
- 13: coupling layer
- 14: acoustic source component
- 14a: acoustic diffractive element
- 14a.1: diffractive layer
- 14a.2: diffractive layer
- 14b: ultrasonic transducer
- 15: fluid control component
- 16: channel portion
- 16a: further channel portion
- 16b: other channel portion
- 17: merging portion
- 18: dispensing channel portion
- 19: outlet
- 20: dispenser
- 22: fluid conduit
- 24: extruder component
- 30: positioning device
- 100: apparatus

## Claims

1. Device (10) for manipulating particles (1), preferably microparticles, in a flow of a working medium (2), preferably hydrogel, the device (10) comprising:
a, preferably sleeve-like, mounting portion (12) for mounting the device (10) to a fluid conduit (22), preferably a fluid pipe, for guiding the flow of the working medium (2); and
an acoustic source component (14) connected to the mounting portion (12) and configured for emitting an acoustic holographic interference field (3) manipulating the particles (1) in the flow of the working medium (2).

2. Device (10) according to claim 1, wherein the acoustic source component (14) comprises:
an acoustic diffractive element (14a), preferably an acoustic transmission hologram, wherein preferably the acoustic diffractive element (14a) comprises at least two diffractive layers (14a.1, 14a.2) for determining a shape of the acoustic holographic interference field (3); and/or
a phased array transducer and a control unit configured to control the phased array transducer to emit the acoustic holographic interference field (3).

3. Device (10) according to claim 1 or 2, wherein:
the mounting portion (12) and/or the device (10) are/is configured for being mounted onto the fluid conduit (22); and/or
the mounting portion (12) forms an annular opening for inserting the fluid conduit (22) therein; and/or
the mounting portion (12) is configured for, preferably concentrically, surrounding an outer surface of the fluid conduit (22); and/or
the mounting portion (12) forms a tapered, preferably conical, recess for accommodating the fluid conduit (22), preferably by clamping the fluid conduit (22).

4. Device (10) according to one of the preceding claims, further comprising:
a, preferably flexible, sealing element arranged at the mounting portion (12), preferably for sealing a joint between the fluid conduit (22) and the mounting portion (12).
a locking element for securing the device (10) to the fluid conduit (22), and preferably for preventing an unintentional removal of the device (10) from the fluid conduit (22).

5. Device (10) according to one of the preceding claims, wherein:
the acoustic source component (14) comprises a cylindrical and/or tubular ultrasonic transducer, preferably a piezo tube actuator; and/or
the acoustic source component (14) comprises a, preferably central, through hole, preferably for passing the flow of the working medium (2) through the acoustic source component (14); and/or
the acoustic source component (14) comprises a concave, cylindrical, and/or closed, preferably circumferentially closed, emitter surface; and/or
the acoustic source component (14) is arranged for emitting the acoustic holographic interference field (3) in a direction perpendicular to a flow direction of the flow of the working medium (2).

6. Device (10) according to one of the preceding claims, wherein:
the acoustic source component (14) comprises a conical and/or frustum-like ultrasonic transducer, preferably a truncated conical shell with a piezoelectric layer; and/or
the acoustic source component (14) comprises a, preferably central, through-hole with a varying, preferably tapered, cross-section; and/or
the acoustic source component (14) comprises a conical and/or tilted emitter surface; and/or
the acoustic source component (14) is arranged for emitting the acoustic holographic interference field (3) tilted with respect to a flow direction of the flow of the working medium (2).

7. Device (10) according to one of the preceding claims, wherein:
the acoustic source component (14) comprises at least one, preferably non-curved, ultrasonic plate transducer, and preferably an acoustic transmission hologram coupled to the at least one ultrasonic plate transducer.

8. Device (10) according to one of the preceding claims, further comprising:
a, preferably straight, channel portion (16) configured for receiving and guiding the flow of the working medium (2), wherein preferably the channel portion (16):
is at least partially formed by a surface of the acoustic source component (14); and/or
is surrounded, preferably coaxially surrounded, by the acoustic source component (14); and/or
is fluidically connectable to the fluid conduit (22).

9. Device (10) according to claim 8, further comprising:
a further channel portion (16a) configured for receiving and guiding a further flow of a further working medium (2a), preferably with further particles (1a); and
a merging portion (17), where the further channel portion (16a) joins the channel portion (16).

10. Device (10) according to claim 9, wherein:
the acoustic source component (14) is configured to surround, preferably circumferentially surround, the merging portion (17); and/or
the acoustic source component (14) is configured to focus the acoustic holographic interference field (3) into the merging portion (17); and/or
the merging portion (17) has an increased cross-section compared to the channel portion (16) and the further channel portion (16a).

11. Device (10) according to claim 9 or 10, wherein:
the device (10) comprises a further mounting portion (12a) for mounting the device (10) to a further fluid conduit (22a) and the further channel portion (16a) is fluidically connectable to the further fluid conduit (22a); and/or
the device (10) comprises a fluid control component (15) configured to selectively allow or disallow a fluid flow in the channel portion (16) and the further channel portion (16a); and/or
the device (10) comprises a dispensing channel portion (18) fluidically connecting the merging portion (17) to an outlet (19) of the device (10); and/or
the device (10) comprises another channel portion (16b) configured for receiving and guiding another flow of another working medium (2b), wherein preferably:
the other working medium (2b) comprises other particles (1b); and/or
the other channel portion (16b) joins the merging portion (17).

12. Dispenser (20), preferably print head, for dispensing a working medium (2) with particles (1), preferably microparticles, therein, comprising:
a fluid conduit (22), preferably a fluid pipe, for guiding a flow of the working medium (2), wherein preferably the fluid conduit (22) is orientated along the direction of gravity; and
a device (10) according to any of claims 1 to 10, wherein the device (10) is mounted to the fluid conduit (22) for manipulating the particles (1) in the flow of the working medium (2).

13. Dispenser (20) according to claim 12, wherein:
the mounting portion (12) has an inner contour, which is adapted to an outer contour of the fluid conduit (22); and/or
the mounting portion (12) is mounted to an end portion of the fluid conduit (22) or the mounting portion (12) is mounted onto the fluid conduit (22), preferably such that the fluid conduit (22) ranges through the entire mounting portion (12); and/or
the dispenser (20) further comprises a, preferably fluid, acoustic coupling layer (13) arranged between the mounting portion (12) and the fluid conduit (22) and preferably for transmitting sound waves between the mounting portion (12) and the fluid conduit (22).

14. Apparatus (100), preferably 3D printer, for fabricating structures of a working medium (2) with particles (1), preferably microparticles, on a substrate (4), the apparatus (100) comprising:
a dispenser (20) according to one of the claims 12 or 13; and
a positioning device (30) configured for changing a relative position of the dispenser (20) to the substrate (4), preferably in three spatial directions.

15. Method for manipulating particles (1), preferably mircoparticles, in a, preferably continuous, flow of a working medium (2), preferably by using the device (10) according to one of the claims 1 to 11, and/or the dispenser (20) according to one of the claims 12 or 13, and/or the apparatus (100) according to claim 14, the method comprising:
providing the flow of the working medium (2), preferably by an extruder component (24); and
emitting an acoustic holographic interference field (3) by an acoustic source component (14) to the flow of the working medium (2) for manipulating the particles (1) in the working medium (2).

16. Method according to claim 15, wherein:
the particles (1) comprise at least one of the following components: a biological cell, a stem cell, a spheroid, an organoid, a tissue fragment, a biomolecule, a bacterium, a virus, a gel bead, a vesicle, a tissue scaffold material, a lipid drop, a hydrogel material, a macromolecule, a polymer, a ceramic particle, a metallic particle, a metal flake, a glass sphere, and a carbon fibre; and/or
the working medium (2) is hydrogel and/or water.

17. Method according to claim 15 or 16, wherein the step of emitting comprises:
emitting the acoustic holographic interference field (3) in a pulsed manner, preferably for structuring an arrangement of the particles (1) along a flow direction of the flow of the working medium (2); and/or
generating the acoustic holographic interference field (3) during an emission period, preferably for assembling the particles (1) to an agglomerate (5), and interrupting the generation of the acoustic holographic interference field (3) during an idle period, preferably for discharging the assembled agglomerate (5).

18. Method according to one of the claims 15 to 17, further comprising:
merging the flow of the working medium (2) and a further flow of a further working medium (2a), preferably comprising further particles (1a), to form a flow of a composite medium,
wherein the step of emitting comprises:
emitting the acoustic holographic interference field (3) to the flow of the composite medium for manipulating the particles (1) in the flow of the composite medium.

19. Method according to one of the claims 15 to 18, wherein:
the working medium (2) differs from the further working medium (2a); and/or
the further working medium (2a) comprises further particles (1a), wherein preferably the particles (1) and the further particles (1a):
respond differently to acoustic, electric and/or magnetic forces; and/or
differ in size and/or material; and/or
each include different biological cell lines.

20. Method according to one of the claims 15 to 19, further comprising:
dispensing the working medium (2) with the manipulated particles (1) on a substrate (4) by a dispenser (20), wherein preferably:
the step of dispensing comprises dispensing the working medium (2) with the manipulated particles (1) in a base layer, and preferably dispensing a plurality of subsequent layers on the base layer for layerwise forming a three-dimensional object; and/or
subjecting the dispensed working medium (2) with the manipulated particles (1) to a fixation, wherein preferably the fixation is a result of biological growth, a timed reaction and/or caused by an external trigger, e.g. thermal energy, irradiation, and/or a fixation agent.
